# EUROPEAN PATENT APPLICATION

(11) **EP 4 270 408 A1**
(43) Date of publication of application: **01.11.2023**
(21) Application number: 22209019.3
(22) Date of filing: 23.11.2022
(51) Int. Cl.: G16H 20/30

(54) **METHOD AND SYSTEM FOR GENERATING EXERCISE PRESCRIPTION BASED ON MULTI-LABEL SCREENING AND DIFFICULTY CLASSIFICATION**

(30) Priority: 27.04.2022 CN 202210447247
(71) Applicant: Chengdu Shangyi Information Technology Co., Ltd., Chengdu, Sichuan 610000 (CN)
(72) Inventor: CHEN, Xi, Chengdu, 610000 (CN); YANG, Fengming, Chengdu, 610000 (CN); ZENG, Ling, Chengdu, 610000 (CN); CHEN, Youlin, Chengdu, 610000 (CN); LEI, Zhen, Chengdu, 610000 (CN)
(74) Representative: M. Zardi & Co S.A.

(57) **Abstract**

The present disclosure relates to a method and system for generating an exercise prescription based on multi-label screening and difficulty classification. The method includes: obtaining movement data from a database; classifying the movement data according to different effects of different exercises on an cardiopulmonary function; obtaining results of an exercise test of a patient; determining an exercise intensity of the patient according to the results of the exercise test; matching the exercise intensity with the classified movement data to obtain a movement data level corresponding to the exercise intensity; and generating a corresponding exercise prescription according to the movement data level. The method can automatically generate the corresponding exercise prescription according to different conditions of patients.

## Description

### TECHNICAL FIELD

The present disclosure relates to the technical field of exercise rehabilitation, and in particular, to a method and system for generating an exercise prescription based on multi-label screening and difficulty classification.

### BACKGROUND

With the increasing population aging in China, more and more people are suffering from chronic diseases in recent years, and the current situation of uneven distribution of medical resources is still difficult to improve in the short term. How to effectively allocate medical resources, reduce the workload of medical staff, and improve the work efficiency of medical staff is an important means to improve the quality of medical services and improve the quality of life of the people in the future.

Rehabilitation therapy is in great demand in the fields of endocrine diseases, cardiopulmonary diseases, chronic pain, orthopedic surgery, and tumor rehabilitation. However, there is a huge talent gap for rehabilitation therapists, and effective rehabilitation prescriptions also rely on personal experience of rehabilitation therapists or rehabilitation doctors, and require a lot of time and effort to prescribe. How to quickly issue rehabilitation prescriptions for different types of diseases, different difficulties, and different types of movements according to specific conditions of patients is an effective method to improve the work efficiency of medical staff.

The exercise prescriptions are mainly issued for people with diseases or the general population who need exercise. The exercise prescriptions include a certain number of rehabilitation movements, which are based on the results of relevant exercise tests. Taking cardiac rehabilitation as an example: the diagnosis of the disease, the results of the cardiopulmonary endurance test, exercise habits, and the age jointly determine the type of exercise, the exercise intensity, the exercise time, and the exercise frequency. The corresponding movement prescriptions are then matched according to these exercise-related factors, but currently, the movement prescriptions are mainly based on fixed exercise templates, such as: swimming, walking, tai chi, rope skipping, jogging, and stepping in place. Even some systems do not include signed related exercise tests to evaluate the exercise ability of the crowd, and only require users to choose exercise prescriptions independently. This type of fixed exercise prescription has the following disadvantages:
1. Excessive exercise may lead to exercise injury.
2. Insufficient exercise may lead to ineffective exercise.
3. Long-term and repetitive movements may cause excessive load to concentrate on local joints and cause j oint damage.
4. The fixed form of exercise stimulates the whole body unevenly, which may lead to muscle strength imbalance in the long term.
5. The same form of exercise may cause a decline in exercise compliance due to lack of freshness.
6. After the patient achieves the exercise goal, the doctor needs to re-evaluate and manually edit a new and appropriate exercise prescription.
7. For patients who already have pain, improper exercise type or exercise intensity can easily provoke pain.

The current exercise prescription system mainly exists in the form of templates, and the exercise content is fixed. Doctors need to manually edit and adjust exercise prescriptions. The editing cost of personalized exercise prescriptions is very high. In addition, because the template is fixed, the movement intensity in the same template is specific, and the movement content is fixed. It is impossible to switch the movement intensity and movement content according to the condition of the patients.

### SUMMARY

An objective of the present disclosure is to provide a method and system for generating an exercise prescription based on multi-label screening and difficulty classification, which can automatically generate the corresponding exercise prescription according to different conditions of patients.

In order to achieve the above objective, the present disclosure provides the following technical solutions.

A method for generating an exercise prescription based on multi-label screening and difficulty classification includes:
obtaining movement data from a database;
classifying the movement data according to different effects of different exercises on a cardiopulmonary function;
obtaining results of an exercise test of a patient;
determining an exercise intensity of the patient according to the results of the exercise test;
matching the exercise intensity with the classified movement data to obtain a movement data level corresponding to the exercise intensity; and
generating a corresponding exercise prescription according to the movement data level.

Optionally, items of the exercise test may include a 3-minute step test, a 2-minute step-in-place test, a resting heart rate test, a 30-second sit-to-stand test, a sit-to-stand-and-walk test, and a 6-minute walking test.

Optionally, the movement data may be obtained by the following modes:
classifying movements according to characteristics of the movements; and
storing the classified movement data in the database.

Optionally, the characteristics of the movements may include parts, types, instruments, body areas, movement intensity classifications, body positions, left and right, and movement patterns.

Optionally, the exercise prescription may include functional exercise, warm-up exercise, whole-body exercise, cool down exercise, and stretching exercise.

A system for generating an exercise prescription based on multi-label screening and difficulty classification includes:
a first data obtaining module, configured to obtain movement data from a database;
a classification module, configured to classify the movement data according to different effects of different exercises on an cardiopulmonary function;
a second data obtaining module, configured to obtain results of an exercise test of a patient;
an exercise intensity determination module, configured to determine an exercise intensity of the patient according to the results of the exercise test;
a data matching module, configured to match the exercise intensity with the classified movement data to obtain a movement data level corresponding to the exercise intensity; and
a prescription generating module, configured to generate a corresponding exercise prescription according to the movement data level.

Optionally, items of the exercise test may include a 3-minute step test, a 2-minute step-in-place test, a resting heart rate test, a 30-second sit-to-stand test, a sit-to-stand-and-walk test, and a 6-minute walking test.

Optionally, the movement data may be obtained by the following modes:
classifying movements according to characteristics of the movements; and
storing the classified movement data in the database.

Optionally, the characteristics of the movements may include parts, types, instruments, body areas, movement intensity classifications, body positions, left and right, and movement patterns.

Optionally, the exercise prescription may include functional exercise, warm-up exercise, whole-body exercise, cool down exercise, and stretching exercise.

According to the specific embodiment provided by the present disclosure, the present disclosure has the following technical effects: 1, the exercise test results form an effective correlation with the exercise prescription; 2, the exercise intensity is defined; 3, the time cost of issuing the exercise prescription is reduced, and the intensity is suitable; and 4, the threshold for doctors to use is reduced.

### BRIEF DESCRIPTION OF THE DRAWINGS

To describe the technical solutions in embodiments of the present disclosure or in the prior art more clearly, the accompanying drawings required in the embodiments will be briefly described below. Apparently, the accompanying drawings in the following description show merely some embodiments of the present disclosure, and other drawings can be derived from these accompanying drawings by those of ordinary skill in the art without creative efforts.
FIG. 1 is a flow diagram of a method for generating an exercise prescription based on multi-label screening and difficulty classification according to the present disclosure;
FIG. 2 is a schematic diagram corresponding to classifications according to an embodiment of the present disclosure;
FIG. 3 is a schematic diagram of a logical relationship according to an embodiment of the present disclosure; and
FIG. 4 is a module diagram of a system for generating an exercise prescription based on multi-label screening and difficulty classification according to the present disclosure.

### DETAILED DESCRIPTION OF THE EMBODIMENTS

The technical solutions in the embodiments of the present disclosure will be described below clearly and completely with reference to the accompanying drawings in the embodiments of the present disclosure. Apparently, the described embodiments are merely some rather than all of the embodiments of the present disclosure. All other embodiments obtained by a person of ordinary skill in the art based on the embodiments of the present disclosure without creative efforts shall fall within the protection scope of the present disclosure.

An objective of the present disclosure is to establish an intelligent recommendation channel between an exercise test and an exercise prescription, so as to solve the following problems in the existing system: 1, the exercise test results cannot form an effective correlation with the exercise prescription; 2, the exercise intensity cannot be defined; 3, the time cost of issuing the exercise prescription is high, the movement is single, and the intensity is uneven; and 4, the threshold for doctors to use is high. The solution provided by the present disclosure can automatically generate the corresponding exercise prescription according to different conditions of patients.

To make the above-mentioned objective, features, and advantages of the present disclosure clearer and more comprehensible, the present disclosure will be further described in detail below in conjunction with the accompanying drawings and specific embodiments.

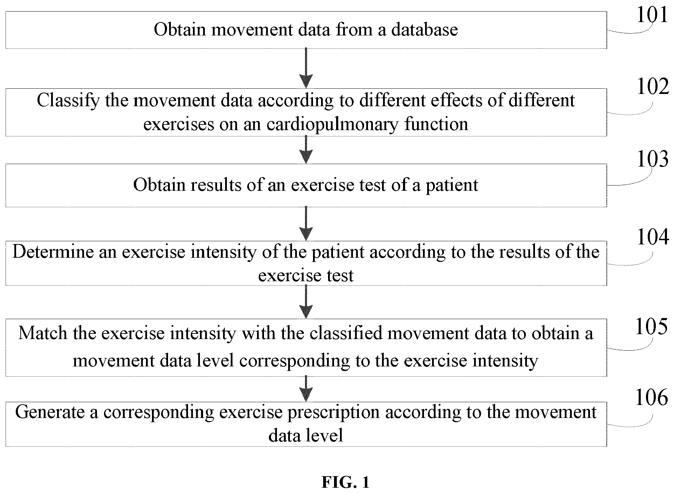

FIG. 1 is a flow diagram of a method for generating an exercise prescription based on multi-label screening and difficulty classification according to the present disclosure. As shown in FIG. 1, the method for generating an exercise prescription based on multi-label screening and difficulty classification includes the following steps.

Step 101: movement data is obtained from a database.

The premise of the whole exercise prescription recommendation system is to have a set of backgrounds containing a sufficient number of movements, which is called a movement database. Subsequent technical solutions can only be realized on the basis of a movement database with a rich number of movements.

Step 102: the movement data is classified according to different effects of different exercises on a cardiopulmonary function.

The human heart rate will increase during exercise, but different body positions, different participating parts, and different exercise movements have different effects on the increase of the heart rate. According to the effects of different movements on the human heart rate, the difficulty of the movements is sorted. The movements are classified into 5 levels according to different effects of different exercises on a cardiopulmonary function. Level 1 is mainly used for bedridden and critically ill patients with poor physical activity. Level 2 is mainly suitable for people with certain bedside mobility. Levels 3 and 4 are suitable for low-risk patients and the general population with different levels of physical activity. Levels 4 and 5 are for the general population with higher activity level needs. The whole movement classification logic is shown in the following table:

| | Movement type | Heart rate increase (for the average person) |
|---|---|---|
| Level 1 | Breathing, local body participation in activities | Barely |
| Level 2 | Seated/partial weight-bearing, most of the body involved in the activity | Slight increase |
| Level 3 | Full weight-bearing, no physical activity requiring strength | Obvious increase |
| Level 4 | Extensive, physical activity requiring strength | Significant increase |
| Level 5 | Physical activity requiring greater strength | Close to the limit |

By classifying the movements, it is helpful for doctors to quickly locate movements with appropriate exercise intensity according to the actual physical condition and cardiopulmonary function of the patient.

Step 103: results of an exercise test of a patient are obtained.

Common test items include a 3-minute step test, a 2-minute step-in-place test, a resting heart rate test, a 30-second sit-to-stand test, a sit-to-stand-and-walk test, and a 6-minute walking test.

Step 104: an exercise intensity of the patient is determined according to the results of the exercise test.

Step 105: the exercise intensity is matched with the classified movement data to obtain a movement data level corresponding to the exercise intensity.

Simplification (to 3 levels) and matching are performed according to the common movement intensity (a total of 3 levels) corresponding to the exercise intensity classification and the classification of the exercise test results. In order to correspond to the effect of the exercise heart rate, the evaluation results classified into 7 levels or 5 levels are combined into 3 levels and the actual exercise intensity is matched with the actual exercise intensity levels 2, 3, and 4.

An example of correlation between the resting heart rate is as follows.

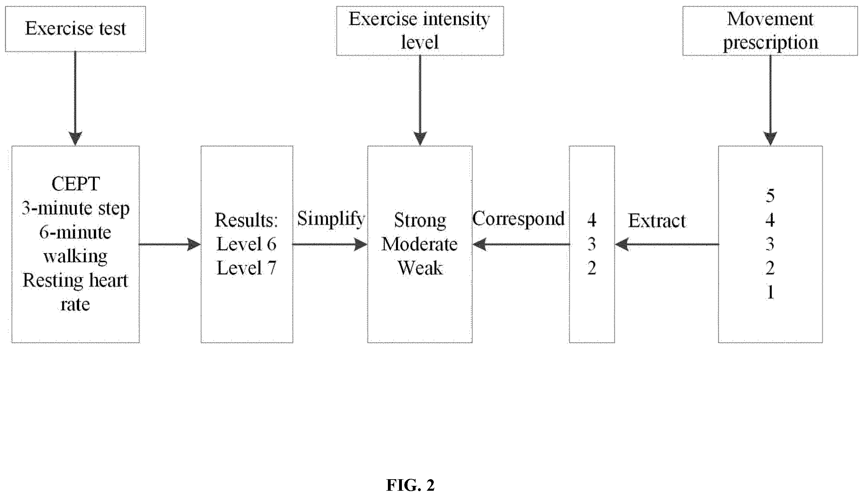

The new "strong (54-65), moderate (66-73), and weak (74 and above)" classifications of the resting heart rate are correlated with "4, 3, 2" in the exercise intensity classification. The aerobic exercise movements suitable for people with strong (athlete and excellent) resting heart rate can correspond to the relevant movements in "intensity 3 and 4". The aerobic exercise movements suitable for people with moderate (good and above average) resting heart rate can correspond to the relevant movements in "intensity 3". The aerobic exercise movements suitable for people with weak (average, below average, and poor) resting heart rate can correspond to the relevant movements in "intensity 2 and 3". This solution can effectively correlate the results of the cardiopulmonary function exercise test with the exercise prescription, such that the exercise test and the exercise prescription can form a corresponding relationship according to the intensity. Taking a patient with heart failure as an example: the results of the resting heart rate evaluation are: "very poor", the results are simplified and classified, the appropriate exercise intensity level for this patient is: "weak", and the corresponding exercise level in the existing exercise prescription is: "2". The classification correspondence is shown in FIG. 2.

Step 106: a corresponding exercise prescription is generated according to the movement data level.

The exercise prescription is a collection of exercise movements for a specific target group and a specific exercise category screened out by a specific formula. In the above example, according to the evaluation results, the heart failure patient can safely perform the relevant exercise with the exercise prescription level 2.

The screening process is as follows: for the data in the movement database, the movements will be classified into multi-dimensional labels according to the characteristics of the movements. The movement characteristics include a total of 8 label dimensions: parts, types, instruments, body areas, movement intensity classifications, body positions, left and right, and movement patterns. Taking stepping in place as an example, according to the above 8 label dimensions, it can be marked as: legs; aerobic; bare hands; whole body; standing position; bilateral; and standing whole body. All movements of the whole movement library are managed and defined in a unified manner in 8 dimensions according to the movement characteristics. In a sufficient number of movement libraries, the multi-label "and/or" inclusion logic and the "and/or" inversion exclusion logic are used, and the movement library screening system of the characteristic label can be used to write the screening formula, and screen out a collection of exercise movements for a specific goal and a specific category.

The screening process can be set as: excluding movements with a part A (or B or C or D) and selecting the intersection of movements containing a type: 1 (or 2 or 3 or 4). Personalized editing can be performed on the system of the present disclosure, which ensures the possibility of intelligent recommendation.

Common exercise programs are classified into a total of 5 modules: functional exercise, warm-up exercise, whole-body exercise, cool down exercise, and stretching exercise. According to the different exercise purposes of each module, the above-mentioned background labeling system needs to be used to screen out different movement collections. On the basis of ensuring safety and effectiveness, an appropriate number of exercises are randomly grabbed from the movement collection to form differentiated exercise prescriptions, which ensures the diversity and scientificity of exercise prescriptions.

Example: according to the recommendations on aerobic exercise prescription for healthy elderly people in the ACSM's Guidelines for Exercise Testing and Prescription by American College of Sports Medicine, it can be summarized as follows: any form of exercise that does not exert excessive pressure on the bones, a combination of moderate-intensity and vigorous-intensity physical activity 3-5 days a week, recommendations of gradually increasing the exercise intensity, avoidance of falling, and requirements of resistance exercise. According to the above recommendations, the basic movement principles of aerobic exercise for the elderly are summarized as: moderate and above exercise intensity, avoidance of falling, avoidance of jumping, and encouragement of whole-body exercise. According to the above movement principles, the movements in the movement library are screened out by formulas.

Disassembled screening formula = type: aerobic and inversion: (movement pattern: (jumping or squatting or lunge squatting or side lunge or deadlift)) and body position: standing position and movement intensity classification: 3 and left and right: bilateral and movement pattern: standing whole body.

According to the logic of this screening formula, a total of 31 movements that conform to this screening logic are screened out from the movement database, which means that there are 31 movements that meet the requirements of the exercise principles of this particular exercise group. Considering that the number of exercises in the exercise prescription for the elderly should not be too many, in order to reduce the difficulty of exercise, the present disclosure can extract a total of 6 movements, classify them into two groups to form an aerobic exercise prescription, and regularly replace the corresponding exercise prescription movements. In summary, the safety, diversity and fun of aerobic exercise prescriptions for the elderly can be guaranteed.

This screening formula can be edited in the system of the present disclosure, so different movement screening formulas can be output for aerobic prescriptions for different healthy people. For different exercise modules (such as stretching after exercise), the screening formulas of different movement modules can also be output by re-editing the screening formula. Finally, the screening formulas can be combined to form a set of exercise prescriptions including functional exercise, warm-up exercise, whole-body exercise, cool down exercise, and stretching exercise.

Disassembled screening formula for stretching = type: physical fitness and type: flexibility and body position: standing position.

In summary, the present disclosure realizes full-automatic recommendation from evaluation to the exercise prescription. The recommended exercise prescription is based on exercise movement classification logic, rich movement library, and movement label management, and combined with the matching of exercise test result classification and exercise movement classification, the closed-loop insurance of the whole-process intelligent exercise prescription from the exercise evaluation to the exercise prescription can be realized, and intelligent automatic recommendation of the exercise prescription is realized.

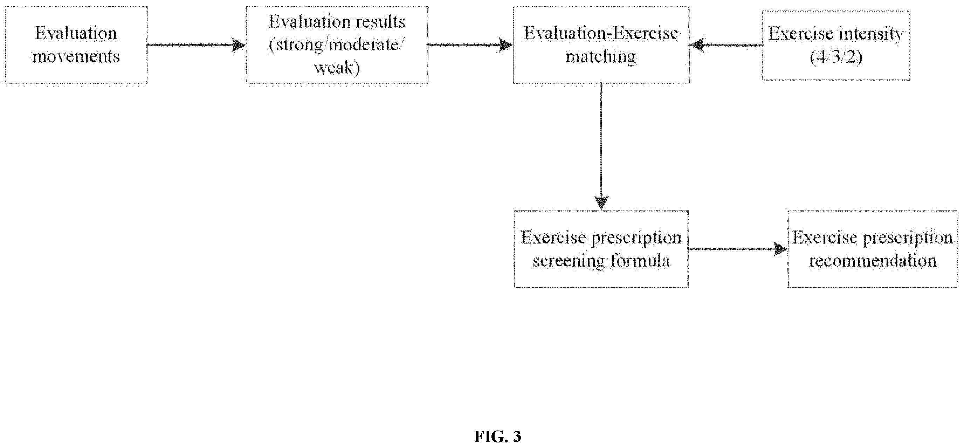

FIG. 3 shows the logic of the whole recommendation process. After the patient completes the relevant exercise evaluation, and an evaluation result (strong-moderate-weak) is generated. Then, evaluation-exercise matching is conducted based on the evaluation result and exercise intensity (4/3/2). Finally, a corresponding exercise prescription result is generated according to the corresponding exercise prescription screening formula.

The present disclosure has the following invention points:
1. Intensity classification method for exercise movements is as follows.
   The present disclosure comprehensively considers the following contents: body position changes of movements: lying position, sitting position, and standing position; movement instrument: elastic band and foam roller; movement type: mobility, flexibility, strength, stability, and balance; other elements of movement. All dimensions of movements are integrated, and the movement intensity is classified into 5 levels, which can screen out movements faster and customize exercise prescriptions.
2. The test intensity is linked to the correlation of test results.
   The corresponding exercise intensity is found by comprehensively analyzing the result classification of relevant cardiopulmonary exercise tests. The correlation between the test and exercise is established, the specific exercise intensity can be obtained by knowing the exercise test result, and combined with the invention point 1, the appropriate exercise movement can be identified faster.
3. Multi-label screening movement recommendation is as follows.
   According to the components of the actual exercise prescription, the exercise intensity level is first determined, and then sufficient movements are comprehensively included/reversely excluded from labels such as body positions, instruments, types, left and right, and parts, and then related diseases, age, and pain that are not applicable are reversely excluded. Finally, a suitable collection of exercise prescription movements is screened out. This enables doctors to edit the label formula at the terminal of the movement library management system, and gradually and accurately find the required movement collection.
4. The full-automatic recommendation from evaluation to the exercise prescription is realized.

Comprehensive use of the invention points 1, 2, and 3 forms a complete automatic exercise prescription recommendation process from exercise evaluation to the exercise prescription. Therefore, doctors and users do not need to manually edit the exercise prescription through the label, but only need to confirm or edit the prescription which is automatically recommended.

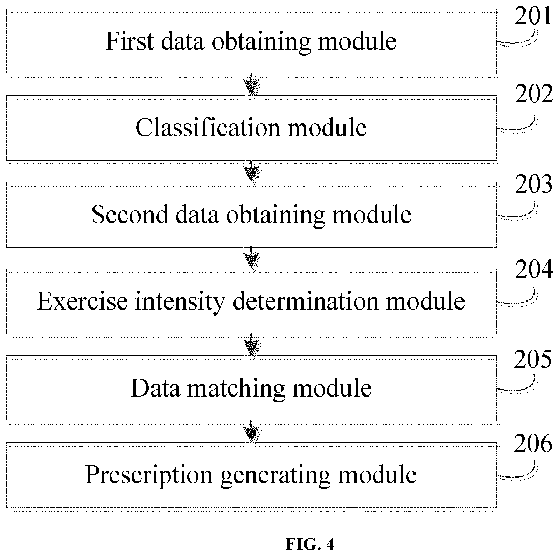

In addition, the present disclosure further provides a system for generating an exercise prescription based on multi-label screening and difficulty classification, as shown in FIG. 4, including: a first data obtaining module, a classification module, a second data obtaining module, an exercise intensity determination module, a data matching module, and a prescription generating module.

The first data obtaining module 201 is configured to obtain movement data from a database.

The classification module 202 is configured to classify the movement data according to different effects of different exercises on a cardiopulmonary function.

The second data obtaining module 203 is configured to obtain results of an exercise test of a patient.

The exercise intensity determination module 204 is configured to determine an exercise intensity of the patient according to the results of the exercise test.

The data matching module 205 is configured to match the exercise intensity with the classified movement data to obtain a movement data level corresponding to the exercise intensity.

The prescription generating module 206 is configured to generate a corresponding exercise prescription according to the movement data level.

The system provided by the present disclosure can allow professionals to edit the label formula, and the system will automatically recommend personalized exercise prescription video guidance according to the label formula. Users at the doctor client only need to confirm the prescription through the doctor client, and do not need to screen out the movement assembly course according to the label.

The present disclosure also has the following technical effects:

The present disclosure classifies the exercise intensity of the exercise movements according to the actual intensity of the exercise movements, realizes differentiated classification according to the exercise intensity, and then can control the exercise intensity in the exercise prescription relatively quantitatively.

The present disclosure correlates the exercise movement classification with the exercise test result classification, such that the exercise intensity corresponding to the evaluation result can be directly correlated with the exercise intensity of the exercise prescription, and the exercise that intelligently matches the corresponding exercise intensity after evaluation can be realized.

The present disclosure performs label management from multiple dimensions according to the characteristics of each exercise movement, including exercise intensity, body position, and type. According to the components of the exercise prescription, the label screening system is used to screen out and combine the composition of random and diversified random exercise prescriptions with uniform intensity.

Each embodiment of the present specification is described in a progressive manner, each embodiment focuses on the difference from other embodiments, and the same and similar parts between the embodiments may refer to each other. Since the system disclosed in an embodiment corresponds to the method disclosed in another embodiment, the description is relatively simple, and reference can be made to the method description.

Specific examples are used herein to explain the principles and embodiments of the present disclosure. The foregoing description of the embodiments is merely intended to help understand the method of the present disclosure and its core ideas; besides, various modifications may be made by a person of ordinary skill in the art to specific embodiments and the scope of application in accordance with the ideas of the present disclosure. In conclusion, the content of the present description shall not be construed as limitations to the present disclosure.

## Claims

1. A method for generating an exercise prescription based on multi-label screening and difficulty classification, comprising:
obtaining movement data from a database;
classifying the movement data according to different effects of different exercises on a cardiopulmonary function;
obtaining results of an exercise test of a patient;
determining an exercise intensity of the patient according to the results of the exercise test;
matching the exercise intensity with the classified movement data to obtain a movement data level corresponding to the exercise intensity; and
generating a corresponding exercise prescription according to the movement data level.

2. The method for generating an exercise prescription based on multi-label screening and difficulty classification according to claim 1, wherein items of the exercise test comprise a 3-minute step test, a 2-minute step-in-place test, a resting heart rate test, a 30-second sit-to-stand test, a sit-to-stand-and-walk test, and a 6-minute walking test.

3. The method for generating an exercise prescription based on multi-label screening and difficulty classification according to claim 1, wherein the movement data is obtained by the following modes:
classifying movements according to characteristics of the movements; and
storing the classified movement data in the database.

4. The method for generating an exercise prescription based on multi-label screening and difficulty classification according to claim 3, wherein the characteristics of the movements comprise parts, types, instruments, body areas, movement intensity classifications, body positions, left and right, and movement patterns.

5. The method for generating an exercise prescription based on multi-label screening and difficulty classification according to claim 1, wherein the exercise prescription comprises functional exercise, warm-up exercise, whole-body exercise, cool down exercise, and stretching exercise.

6. A system for generating an exercise prescription based on multi-label screening and difficulty classification, comprising:
a first data obtaining module, configured to obtain movement data from a database;
a classification module, configured to classify the movement data according to different effects of different exercises on a cardiopulmonary function;
a second data obtaining module, configured to obtain results of an exercise test of a patient;
an exercise intensity determination module, configured to determine an exercise intensity of the patient according to the results of the exercise test;
a data matching module, configured to match the exercise intensity with the classified movement data to obtain a movement data level corresponding to the exercise intensity; and
a prescription generating module, configured to generate a corresponding exercise prescription according to the movement data level.

7. The system for generating an exercise prescription based on multi-label screening and difficulty classification according to claim 6, wherein items of the exercise test comprise a 3-minute step test, a 2-minute step-in-place test, a resting heart rate test, a 30-second sit-to-stand test, a sit-to-stand-and-walk test, and a 6-minute walking test.

8. The system for generating an exercise prescription based on multi-label screening and difficulty classification according to claim 6, wherein the movement data is obtained by the following modes:
classifying movements according to characteristics of the movements; and
storing the classified movement data in the database.

9. The system for generating an exercise prescription based on multi-label screening and difficulty classification according to claim 8, wherein the characteristics of the movements comprise parts, types, instruments, body areas, movement intensity classifications, body positions, left and right, and movement patterns.

10. The system for generating an exercise prescription based on multi-label screening and difficulty classification according to claim 6, wherein the exercise prescription comprises functional exercise, warm-up exercise, whole-body exercise, cool down exercise, and stretching exercise.
